# EUROPEAN PATENT APPLICATION

(11) **EP 4 745 223 A1**
(43) Date of publication of application: **20.05.2026**
(21) Application number: 24315525.6
(22) Date of filing: 18.11.2024
(51) Int. Cl.: C12M 3/00, C12M 1/32, C12M 1/12, C12M 1/00, C12M 1/42

(54) **BIOREACTOR AND PROCESS TO PRODUCE OSTEOARTICULAR ORGANOIDS**

(71) Applicant: Université Paris Cité, 75006 Paris (FR); Centre National de la Recherche Scientifique, 75016 Paris (FR); Institut National de la Santé et de la Recherche Médicale (INSERM), 75013 Paris (FR); Ecole Nationale Vétérinaire D'Alfort, 94700 Maisons-Alfort (FR); Ecole Centrale de Lyon, 69130 Ecully (FR)
(72) Inventor: Hoc, Thierry, 69260 CHARBONNIERES LES BAINS (FR); Bensidhoum, Morad, 92310 SEVRES (FR); Potier, Esther, 92150 SURESNES (FR); Rouquier, Léa, 75013 PARIS (FR); Petite, Hervé, 75011 PARIS (FR)
(74) Representative: Jorget, Quentin

(57) **Abstract**

Invention concerns a method (1) for producing osteoarticular organoids with a bioreactor (2), the method (1) comprising a mechanical stimulation step (13) that occurs during a feeding step (12). While the feeding step (12) feeds the cells (CLS) with a nutrient fluid (FLD), the stimulation step (13) provide cyclic compressions to the cells (CLS), in order to activate the mechanical characteristics sought for such osteoarticular organoids depending on its own stiffness. The production occurs in a sterile environment and with hypoxic conditions, said metho comprising an oxygen regulation step (14) and a temperature regulation step (15) to finely control the temperature at 37°C during the production phase, i.e. more than 1 month.

## Description

[The technical context of the present invention is that of the production of osteoarticular organoids that can be used to carry out clinical studies or clinical trials. More particularly, the invention relates to a bioreactor for producing such osteoarticular organoids in a particularly efficient and qualitative manner, as well as a method for producing such osteoarticular organoids.

The production of organoids makes it possible to provide in vitro tissues that have some functionalities and properties close to living tissues. Thus, the production of such organoids represents a crucial issue for the development of modern medical techniques. In particular, in the field of regenerative medicine, the development of organoids makes it possible to imagine personalized treatments and gene therapies to restore the functionality of damaged tissues. It is therefore understood that an underlying issue lies in avoiding the use of animal experimentation, which is now increasingly constrained by national legislations.

In the state of the art, the production of kidney, lung or brain organoids is already known. The present invention does not address the problem of the production of such "soft" organoids but on the contrary addresses the production of osteoarticular organoids, such as for example bone tissues, cartilages or intervertebral discs. Such osteoarticular organoids cannot be manufactured by the techniques currently known for "soft" organoids .

In particular, document WO2009010661 A2 is known, which describes a method of producing bone tissues in which cells are deposited on a 3Dscaffold and feeded with a nutritional fluid comprising calcium carbonate. During the culture phase, the cells are maintained in the nutritional fluid, and it is the nutritional fluid which, by its flow, generates shear stresses - and more particularly wall shear stresses - at the level of the cells. The disadvantage of the method implemented in this document lies in its lower efficiency This is mainly due to the fact that the method does not provide the mechanical and biological 3D micro-environment with which the cell is confronted in vivo. Thus, the mechanical load to be applied on the cells by the bioreactor should be controlled through a displacement-driven actuator whose position in finely tuned - typically at the micrometer scale, which is not achievable with the mechanism disclosed in WO2009010661 A2 .

Moreover, in fact, in vivo a cyclic uniaxial compressive load is applied on a daily basis during walking, which is the main stimulus. This periodic load should be perfectly controlled at the micrometre scale, as explained before, which is not achievable with the mechanism disclosed in WO2009010661 A2. Then, as the rigidity of the organoid evolves over time, it is necessary to measure it and to finely control the mechanical load applied by the actuator over the growing period. Unfortunately, the mechanism disclosed in WO2009010661 A2 is not suitable for that.

In addition, from the biological point of view, nutrient quality and hypoxic conditions, which need to be continuously monitored, are essentials part of the biological microenvironment. Unfortunately, the mechanism disclosed in WO2009010661 A2 is not suitable for providing such optimal environment.

The scientific article "An organoid for woven bone" published by Anat Akiva et al. in "Advanced Functionals Material" is also known, and it
The object of the present invention is to propose a new method for producing osteoarticular organoids in order to remediate or resolve at least some of the problems described above, and to lead to further other advantages.

Another aim of the invention is to produce more qualitative osteoarticular organoids.

Another aim of the invention is to be able to maintain mechanical stimulation felt by cells during organoid formation for long periods, typically several weeks.

Another aim of the invention is to reproduce mechanical load on felt by the cells during organoid formation close to those felt by human cells during walking for example.

According to a first aspect of the invention, at least one of the aforementioned objectives is achieved with a method for producing osteoarticular organoids using a bioreactor, the production method comprising the following steps:
- a step of depositing cells onto a scaffold, called the seeding step, in at least one growth well of the bioreactor, the cells being of the type of cells capable of forming an osteoarticular organoid;
- a step of feeding the cells, called the feeding step, by using a nutrient fluid providing each at least one growth well in order to provide a chemical microenvironment to the cells during the formation of the osteoarticular organoid, the feeding step being carried out by a diffusion element of the bioreactor;
- a step of mechanical stimulation of each at least one growth well, called the stimulation step, in order to induce strain constraints felt by the cells during organoid formation, the stimulation step being carried out by a device for applying mechanical load of the bioreactor, called application device.

In the context of the present invention, the cells used for the feeding step are of the type of capable of becoming an osteoarticular organoid. As a non limitative example, such cells could be for example cells of a tissue, or stem cells, or embryonic stem cells, or induced pluripotent stem cells.

In the context of the present invention, the seeding step aims at providing cells on a 3D scaffold as described therebefore, in each of the at least one growth well of the bioreactor. During the seeding step, the cells are seeded onto a deposition substrate of the bioreactor or on a predetermined substrate used with the bioreactor, as a 3D scaffold for example. In a more general way, the deformation substrate is a 3D surface that can be seeded with cells and onto which the cells will grow over the time of the method. Thus, in a more general way, the seeding step comprise a deposition of cell on a substrate - the deposition substrate - on which - directly or indirectly - cells are going to evolve to the organoid. The seeding step is a preliminary step of the method according to the invention that is achieved once and before the feeding step and the stimulation step that are achieved afterward.

In the context of the present invention, the feeding step aims at providing to the cells a chemical microenvironment that are necessary during organoid formation, through the nutrient fluid. According to the invention, the nutrient fluid is provided by the diffusion element of the bioreactor in order to feed each cells in each at least one growth well with such nutrients. Thus, the nutrient fluids flows in each at least one growth well in order to drown the cells into a very efficient media. The bioreactor - and its diffusion element in particular - includes means for make it possible to the nutrient fluid to flow toward each at least one growth well. Optionally, the feeding step and the stimulation step can be simultaneous, or the stimulation step is achieved once the feeding step is over.

In the context of the present invention, the stimulation step is producing a mechanical load on the growing cells located into the at least one growth well. Unlike the known state of the art, the present invention does not provide such mechanical load by the nutrient fluid flow through the bioreactor and the growth wells. Indeed, the present invention provide a mechanical excitation of the overall structure, and more precisely onto or nearby the at least one growth well and the cells. More precisely, as it'll be described with more details hereafter, the stimulation step is achieved via a dedicated application device of the bioreactor, such as an actuator, a piston, a motor or any kind of device that is suitable for producing vibrations, displacements and, in a more general way, a force onto the deposition substrate - a 3D scaffold for example - onto which the cells have been deposited earlier. In a very general way, in the context of the present invention, the application device of the bioreactor is configured for applying some contacts forces to the organoid according to its stiffness.

Thus, the method according to the first aspect of the invention solves the technical problem defined here before in that it makes it possible to produce more efficiently and/or over longer periods of time cells which gradually transform into osteoarticular organoids. Indeed, applying prolonged and repeated mechanical stimulation felt by the cells during organoid formation imply mechano-transduction process .

In the context of the present invention, the mechanical constraints imposed by the application device of the bioreactor are oriented in a direction perpendicular to the deposition substrate, i.e. the 3D scaffold axis, on which the cells have been previously deposited in the at least one growth well of said bioreactor.

The method with respect to the first aspect of the invention advantageously includes at least one of the improvements below, the technical characteristics included in these improvements being considered independently or in combination:
- the feeding step and the stimulation step are carried out in a sterile environment, the at least one growth well and bioreactor providing a sterile environment for the cell growth. For this purpose, the at least one growth well is preferably sealed during the duration of the method;
- the feeding step and/or the stimulation step are carried out under hypoxic conditions. To this purpose, the method comprises a step of regulating an oxygen concentration, called the oxygen regulation step, said oxygen regulation step occurs until the feeding step and the stimulation step are completed. More precisely, during the feeding step and/or the stimulation step, the cells present in each at least one growth well are subjected to an air flow whose oxygen concentration is less than 10%, preferably less than 5%, preferably still equal to or substantially equal to 1.5%. In particular, according to a first embodiment variant particularly suitable for the formation of an osteoarticular organoid of the bone tissue type, the method comprises a step of regulating an oxygen concentration, called the oxygen regulation step, in the nutrient fluid, the oxygen concentration being between 5% and 7%, at least at the start of cell production. According to a second embodiment variant particularly suitable for the formation of an osteoarticular organoid of the cartilage type, the method comprises a step of regulating an oxygen concentration, called the oxygen regulation step, in the nutrient fluid, the oxygen concentration being zero or substantially zero, at least at the start of cell production. According to a third embodiment variant particularly suitable for the formation of an osteoarticular organoid of the intervertebral disc type, the method comprises a step of regulating an oxygen concentration, called the oxygen regulation step, in the nutrient fluid, the oxygen concentration being less than 1.5%, at least at the start of cell production;
- according to a preferred embodiment of the invention, the feeding step is carried out continuously. In other terms, the nutrient fluid flows continuously through each at least one growth well, said flow being invariant or variable during the stimulation step. Thus, the stimulation step and the feeding step are advantageousely simultaneous. Alternatively, according to another embodiment of the invention, the feeding step is carried out discontinuously during seeding periods. The seeding periods have durations of between a few minutes and several hours. In addition, the seeding periods are spaced from each other by periods of inactivity which can last a few minutes to a few hours. Consequently, a seeding ratio is defined as the ratio between the seeding periods and the inactivity periods, the seeding ratio preferably being greater than 1;
- in a very general way, in the context of the present invention, the nutrient fluid used to feed the cells is of the glucose-rich type, i.e. comprising a non-zero glucose concentration. In particular, according to a first embodiment variant particularly suitable for the formation of an osteoarticular organoid of the bone tissue type, the nutrient fluid comprises (i) 5.6 mL of xamethasone at 10 uM, (ii) 5.6 mL of vitamin C at 15 mM, (iii) 1.1 mL of betaGlycero at 1M, (iv) 56.4 mL of decomplemented fetal calf serum, (v) 5 mL of an antibiotic prophylaxis, (vi) 500 mL of alpha minimal essential medium. According to a second embodiment variant particularly suitable for the formation of an osteoarticular organoid of the cartilage type, the nutrient fluid comprises (i) a glucose-rich Dulbecco's modified Eagle's medium, (ii) a universal culture supplement containing insulin, human transferrin and selenious acid,(iii) sodium pyruvate, preferably at a concentration of the order of 110 µg/mL, (iv) proline, preferably at a concentration of the order of 40 µg/mL, (v) dexamethasone, (vi) L-ascorbic acid 2-phosphate, preferably at a concentration of the order of 50 µg/mL, (vii) a transforming growth factor ß1, preferably at a concentration of the order of 10 ng/ml. According to a third embodiment variant particularly suitable for the formation of an osteoarticular organoid of the intervertebral disc type, the nutrient fluid comprises (i) a Dulbecco's modified Eagle's medium, without glucose and having a pH equal to 7.1, preferably at a concentration of the order of 430 mOsm/kg and supplemented with 2 mM of glucose, (ii) 1% of a universal culture supplement containing insulin, human transferrin and selenious acid, (iii) 50 µM of ascorbic acid, (iv) 10-8 M of dexamethasone, (v) a transforming growth factor ß1, preferably at a concentration of the order of 10 ng/mL, (vi) a growth differentiation factor 5, preferably at a concentration of the order of 100 ng/mL;
- in a very general way, the stimulation step is carried out during the seeding step. According to a first embodiment, the stimulation step is carried out throughout the feeding step. This advantageous configuration allows a strong control of the environmental conditions, and more precisely a strong respect of hypoxia. Alternatively, according to a second embodiment, the stimulation step is carried out during one or more cycles of said feeding step, called stimulation cycles. In particular, each at least one stimulation cycle has the same predetermined stimulation duration. As a non-limiting example, the stimulation duration is between a few minutes and a few hours. Particularly advantageously for the production of osteoarticular organoids of the intervertebral disc type, the stimulation duration is of the order of 2 hours;
- the simulation step is carried out periodically according to a predetermined stimulation period. The simulation cycles are spaced apart by rest periods that can last a few minutes to a few hours. Consequently, a simulation ratio is defined as the ratio between the simulation cycles and the rest periods, the simulation ratio preferably being between 1% and 15%, preferably between 2% and 10%, preferably still equal or substantially equal to 9%. This advantageous configuration makes it possible to simulate more precisely the human walk that is daily recommended by the World Health Organization, as 9% corresponds to 2 hours (of stimulation for the cells) per day, while 2% corresponds to 30 minutes (of stimulation for the cells) per day;
- the stimulation step generates a series of mechanical load applied to each at least one growth well, the mechanical load aiming to impose repeated deformation or forces on the cells and/or on the scaffold during organoid formation. In other words, the series of load thus produced is produced during each at least one stimulation cycle. For this purpose, the mechanical load produced during the stimulation step are configured to generate on the cells of each at least one growth well an initial deformation at least equal to 10% for soft organoids, and at least equal to 4% for hard organoids. The deformation is here understood as a relative deformation, obtained trough the ratio of the elongation divided by the length of the scaffold;
- the mechanical load produced during the stimulation step are configured to generate decreasing deformations on the scaffold seeded with cells during a production duration, the deformations induced by the mechanical load at the start of culture being higher than those introduced by the mechanical load at the end of culture. As a non limitative example, at the start of culture, the mechanical load produced during the stimulation step are configured to generate deformations on the cells in the range of 10% and, at the end of culture, the mechanical load produced during the stimulation step are configured to generate deformations on the cells in the range of at least 0,2%;
- between the start amplitude and the end amplitude, the amplitude of the mechanical load produced during the stimulation step varies advantageously in a linear manner. According to a first embodiment variant particularly suitable for the formation of an osteoarticular organoid of the bone tissue type, the mechanical load produced during the first stimulation steps impose a deformation of the cells deposited in the at least one growth well of the order of 4%. According to a second embodiment variant particularly suitable for the formation of an osteoarticular organoid of the cartilage type, the mechanical load produced during the first stimulation steps impose a deformation of the cells deposited in the at least one growth well of the order of 10%. According to a third embodiment variant particularly suitable for the formation of an osteoarticular organoid of the intervertebral disc type, the mechanical load produced during the first stimulation steps impose a deformation of the cells deposited in the at least one growth well greater than 8%;
- the mechanical constraints carried out during the stimulation step are carried out at a determined frequency. The frequency may be invariant during the stimulation step, or it may be variable during said stimulation step. As a non limitative example, the frequency is advantageously comprised between 0.5Hz and 10Hz, preferably between 0.5 Hz and 5 Hz, more preferably equal or substantially equal to 1 Hz. Such frequency range is quite similar to the excitation frequency of bones and muscles during human activities, and more specifically during a walk (about 1 Hz) or during a run (5 to 10 Hz). In a more precise way, and as a non limitative example, the mechanical constraints produced during the stimulation step are produced by a piston movement applied to each at least one growth well, the piston movement transferring a load to a scaffold supporting the cells in each at least one growth well;
- the method comprises a step of regulating a temperature of the nutrient fluid, called the temperature regulation step, to a predetermined set temperature, preferably equal to or substantially equal to 37°. This advantageous specification provide some very well-defined conditions for the cell microenvironment;
- the method according to the invention lasts for a period of at least 2 weeks, preferably at least 8 weeks. Of course, the longer the method, the more functionalized the osteoarticular organoids. Nevertheless, it's very important to maintain a very high level of sterility inside the bioreactor during throughout the experiment.

According to a second aspect, the invention aims at proposing a bioreactor for producing an osteoarticular organoid, the bioreactor comprising means configured to implement the production method according to the first aspect of the invention or any one of its embodiments.

More precisely, the bioreactor's means comprise:
- a frame;
- a culture holder housed in the frame, the culture holder comprising at least one growth well forming a cavity arranged on the culture holder itself, each at least one growth well delimiting a deposition substrate - as well as a 3D scaffold for example - for cells intended to be produced in order to form the osteoarticular organoid;
- an element for diffusing a nutrient fluid in each at least one growth well, called diffusion element;
- a device for applying mechanical load to each at least one growth well, called application device, said application device being configured to generate an axial force directly above each deposition substrate, i.e. the 3D scaffold;
- a control unit configured to drive the application device and the diffusion element.

In the context of the present invention, the frame provides a compact, localised, and protected environment for the culture holder and the cells in the bioreactor. The frame is configured to provide an inner cavity opening towards the top or towards one side of the bioreactor in order to make it possible be able to put the scaffold seeded with cells onto the culture holder in the at least one growth well, in a hypoxic environment. The frame also comprises some retaining elements that are configured to attach the culture holder to said frame.

In the context of the present invention, the culture holder is configured for being mounted inside the frame, and more specifically inside the inner cavity provided by the frame. This advantageous configuration makes it possible to protect the culture holder, and thus the at least one growth well and the cells seeded on the deposition substrate - i.e. the 3D scaffold - of said growth well. The culture holder is configured to be axially inserted into the frame, regarding said frame's inner cavity. The culture holder aims at providing the at least one growth well for the cell's culture. Each growth well is designed as a small cavity housed on the culture holder. As a non limitative example, each growth well as a circular peripherical edge and is also defined with an underlayer providing a flat surface for placing the scaffold. As an example, the width of each growth well is comprised between 2 mm and 10 mm, and its depth is at least equal to 2 mm. Of course, such dimensions can be adjusted depending on the size of the osteoarticular organoids that are sought and to adapt the desired nutrient consumption.As a preferred embodiment, each at least one growth well has a width of 6 mm and a depth of 9 mm.

In the context of the invention, the culture holder holds one or many growth wells, depending on the number of osteoarticular organoids that are intended to be fabricated, with a limit of 8. Every growth well is supported by the culture holder. Each growth well is designed on the same face of the culture holder.

In the context of the present invention, the element for diffusing is configured to make it possible for the nutrient fluid to flow into and on each at least one growth well. The diffusion element is designed to organise the nutrient fluid distribution - i.e. the transportation toward each growth well and the feeding of each growth well.

In the context of the present invention, the application device is configured to impose compression of the scaffold seeded with cells deposited on deposition substrate of each at least one growth well. To his purpose, the application device is arranged above each at least one growth well. In other words, the application device if facing at every growth well in order to force a mechanical deformation felt by the cells during organoid formation. The force is applied by contact of a free extremity of the device onto the scaffold and, more generally, the deposition substrate In a more general way, a force or a displacement is provided by the application device for every cells located into each growth well, as it'll be described in more detail hereafter.

In the context of the present invention, the control unit comprises a microcontroller and/or a printed circuit and/or a microprocessor. Additionally, the control unit also optionally comprises a memory.

The bioreactor with respect to the second aspect of the invention advantageously includes at least one of the improvements below, the technical characteristics included in these improvements being considered independently or in combination:
- the frame is formed of a metallic material, preferably of the type of a good thermal conductor and is not prone to oxidation, such as for example stainless steel. This advantageous configuration allows to establish some good thermal condition inside the bioreactor and to provide some stable thermal conditions along the duration of the organoid formation.
- the frame has a general cylindrical conformation along a specific elongation axis, the culture holder being housed axially in the frame, the application device forming a closing cover at an axial end of the frame. The culture holder is arranged inside the cylindrical conformation, being inserted by a translation movement of the culture holder relatively to the frame. Thus, the cylindrical conformation of the frame acts as a guiding element for the culture holder. This advantageous configuration is important for a well controlled force or displacement application by the application device;
- the at least one growth well of the culture holder forms a concavity arranged along the axial elongation axis of the frame;
- the nutrient fluid diffusion element comprises a diffusion channel arranged in the culture holder and opening onto each at least one growth well. Preferably, the diffusion channel takes the form of a groove arranged in the culture holder. In the case where the culture holder comprises a plurality of growth wells, then the diffusion channel connects each growth well in a serial or star configuration. This advantageous configuration makes it possible to feed with nutrient in a very efficient manner and an homogeneous manner for each growth well. As a non-limitative example, the culture holder is of PEEK material type;
- the flow rate of the nutrient fluid diffusion element is advantageously equal or approximately equal to 0,1 mm.s⁻¹.
- in order to feed each growing cells with the right amount of nutrient, and in the case where the culture holder comprises a plurality of growth wells, then the diffusion channel opens at a level lower than the deposition substrate of each growth well. This advantageous configuration makes it possible for the nutrient fluid that flows onto the diffusion channel to flow into the growth well and toward the cells in a very efficient manner. Then, in the case where the culture holder comprises a plurality of growth wells, then thedeposition substrate of each growth well are successively higher, relative to a flow direction of a nutrient fluid in the diffusion channel. In other words, in the case where there are several growth well, there are arranged in a step configuration, the diffusion channel connecting each adjacent growth well;
- as describer before, the depth and/or a width of the diffusion channel is between 2 mm and 10 mm;
- in a very general way, the feeding step and the stimulation step are carried out in a sterile environment. For this purpose, the bioreactor comprises a membrane for sealing the culture holder, the membrane being configured to seal the culture holder in a sealed manner, i.e. to seal the at least one growth well and the diffusion channel. The membrane is associated with the culture holder, in order to close the diffusion channel and each at least one growth well. The membrane is deformable along the specific elongation axis of the frame. This advantageous configuration makes it possible to transmit the mechanical load provided by the application device to the cells, via the membrane itself. In other words, the membrane is configured to be deformable along the elongation axis of the frame. As a non limitative example, the membrane is of the silicone membrane type;
- regarding a preferred embodiment of the invention, the application device comprises (i) a force generator configured to generate an axial force on the deposition substrate - a 3D scaffold - of each at least one growth well, and (ii) a compression sensor configured to determine the axial force applied by the force generator on the deposition substrate - the 3D scaffold - of each at least one growth well, the control unit controlling the application device according to the axial force measured by the compression sensor. In the context of the present invention, the force generator may be of any type, such as for example a piezoelectric actuator, an electric motor or a cylinder. Advantageously, according to a preferred embodiment of the invention, the force generator is of the type of a pneumatic piston to avoid contamination;
- the application device comprises an adapter coupled to a free end of the force generator, the adapter being configured to transmit the force produced by the axial movement of the force generator to each at least one growth well, a free end of the adapter being associated with each growing well. The adapter has an elongated shape that is suitable with the cylindrical shape of the frame. The adapter is configured to be located inside the frame, as it "connects" the application device to the culture holder. The free end of the adapter is for example in contact with the membrane, or with the deposition substrate - i.e. the 3D scaffold - or the cells itself, or with a levelling pellet to be described hereafter;
- in order to guide the adapter along the elongation axis and perpendicular to the growth wells, the adapter comprises a translational guiding beam along the elongation axis of the frame, the guiding beam collaborating by sliding in a cylindrical opening provided in the frame. Thus, it makes it possible to finely control the force applied by the actuator - through the adapter - on the scaffold seeded with cells surface of each at least one growth well. This advantageous configuration is particularly relevant while the culture holder of the bioreactor comprises more than one growth well. Indeed, it prevents the adapter from tilting regarding the elongation axis and then, providing an equal repartition of the load applied to each growth well. Advantageously, the translational guiding beam elongates parallel to the elongation axis of the bioreactor, i.e. its greater direction, i.e. the elongation axis of the frame. Preferably, the translational guiding beam elongates coaxially to the bioreactor;
- in more details, the adapter comprises at least one terminal finger, each at least one terminal finger being associated with one of the at least one growth well so as to transmit the force produced by the actuator. In other words, the adapter comprises as many terminal fingers as the number of growth wells of the culture holder. Then, each terminal finger has dimensions and shape that makes it possible to collaborate with each growth well placed opposite. The force generator and the adapter are facing the growth wells - and the cells deposited in - regarding the elongation axis of the frame;
- the adapter is formed of a metallic material, such as for example stainless steel;
- in a first variant of realisation, the adapter is attached to the free end of the force generator. Thus, when the force generator is moving away from the deposition substrate, then the adapter is also moving away from the deposition substrate ; and once the force generator gets closer to the deposition substrate, then the adapter is also approaching the deposition substrate. In a second variant of realisation, the adapter is not attached to the free end of the force generator, and the bioreactor comprises an opposite force generator providing a force opposite to the one produced by the force generator. The opposite force generator comprises for example a spring device located between the free end of the adapter and the frame of the bioreactor: when the force generator gets closer to the deposition substrate, then the spring device is pushing against the adapter, but the force provided but the force generator remains greater than the force of the spring device. Once the force generator is moving back, then the force provided by the spring device allows the adapter to move back with the force generator. Optionally, even if the adapter is attached to the force generator, the bioreactor may advantageously comprise such spring device in order to facilitate the backward movement of the force generator and its adapter. The spring device is particularly advantageous in the case where the force generator is of the pneumatic type for example.
- the bioreactor comprises a displacement sensor of the adapter, the displacement sensor being attached with the frame. The displacement sensor is configured to measure the displacement of the adapter and/or of the free extremity of the application device with regard to the frame, in order to transfer information to the control unit about such displacement and makes it possible to compute a load or a pressure applied by said application device. The displacement sensor is preferably of the type of an optical sensor, such as for example a laser distance meter;
- in order to facilitate such measurement, the adapter comprises a lateral flange which extends radially outside the frame, the displacement sensor measuring the displacement of the lateral flange regarding to the frame. Such advantageous configuration makes it possible to attach the displacement sensor outward from the frame and the experiment itself;
- the compression sensor is preferably of the type of a force sensor configured to measure a compression force exerted by a free end of the guide surface of the adapter relative to the frame. For this purpose, the force sensor is located at a lower end of the cylindrical opening of the frame, the free end of the adapter exerting a force on the force sensor proportionally to the displacement of the force generator. Then, the axial force applied to the deposition substrate - i.e. the 3D scaffold - seeded with cells in each at least one growth well is thus derived from the stiffness of the organoid evolving during its formation. The stiffness is obtained by calculating the ratio between the force measured by the compression sensor and the elongation of the force generator and measured by the displacement sensor;- the bioreactor comprises at least one levelling pellet arranged in each at least one growth well, above the cells, a height of the at least one levelling pellet being such that it is flush with an upper face of the culture holder. Each levelling pellet acts as an offset to compensate the different heights of each deposition substrate seeded with cells placed in said growth wells, thus the adapter lie on each levelling pellet in a equally and balanced manner. In the case where the culture holder comprises a plurality of growth wells, then the levelling pellets associated with each growth well are chosen so that they are ultimately all substantially at the same height. The at least one pellet is formed from a composite material or a plastic, preferably of the thermoplastic type, such as for example a polyetheretherketone (PEEK).

According to a third aspect, the invention aims at proposing a system for producing an osteoarticular organoid, and the culture system comprising:
- the bioreactor according to the second aspect of the invention or to any one of its embodiments;
- a nutrient fluid storage, the nutrient fluid storage being connected to the diffusion element of the bioreactor by fluid conduits;
- a pump for controlling a flow rate of the nutrient fluid in the diffusion element of the bioreactor;
- an heating system configured to regulate a temperature of the nutrient fluid stored in the nutrient fluid storage at a set temperature, preferably equal to or substantially equal to 37°;
- a hypoxia oven for controlling an oxygen concentration in the bioreactor and in particular at the diffusion element;
- a control unit configured to control the culture system, the control unit being interfaced with the control unit of the bioreactor.

Various embodiments of the invention are provided hereafter, said embodiments being considered according to anyone of their possible combinations, explicitly described or implicitly disclosed.

Other characteristics and advantages of the invention will become highlighted through the description that follows on the one hand, and through several embodiments given as some not limiting examples related to drawings on the other hand, on which :
[Fig.1] illustrates a synoptic diagram of the method according to the first aspect of the invention ;
[Fig.2] illustrates a schematic view of the bioreactor according to the second aspect of the invention.

Of course, the characteristics, the alternatives and the different embodiments of the invention can be associated with each other, according to various combinations, insofar as they are not incompatible or exclusive of each other. In particular, alternatives of the invention may comprise only a selection of characteristics described below, if such selected characteristics is enough to provide a technical advantage or to differentiate the invention from to the state of the prior art.

In particular, the alternatives and the embodiments described can be combined with each other if nothing prevents this combination from a technical point of view.

In the figures and the description, elements that are visible on several figures retain the same reference.

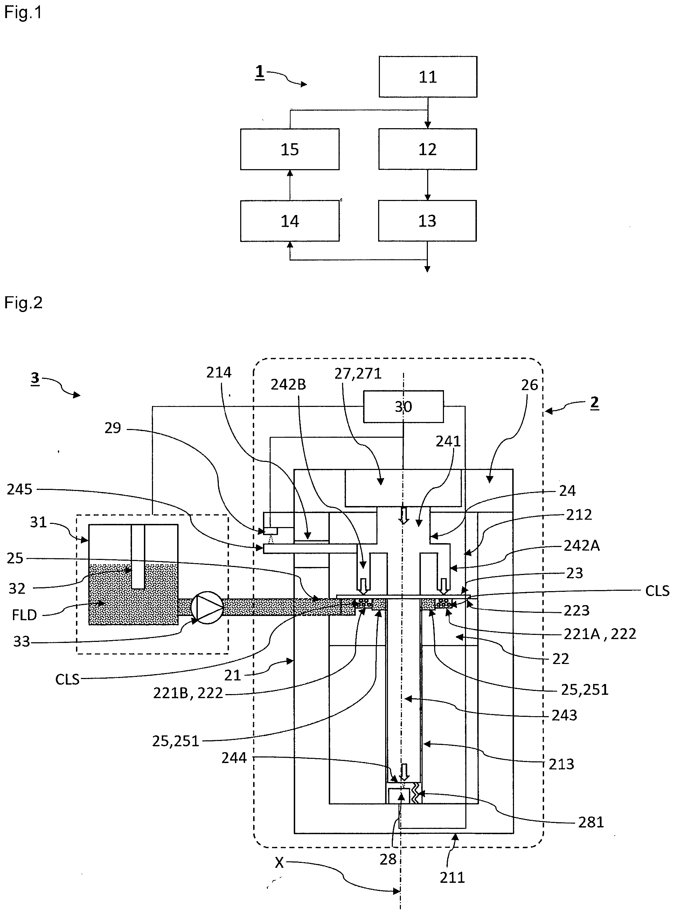

In reference to FIGURE 1, the invention addresses a method 1 for producing osteoarticular organoids using a bioreactor 2, the production method 1 comprising the following steps:
- a step of seeding cells CLS onto a deposition substrate 222, as a 3D scaffold for example, called the seeding step 11, in at least one growth well 221B, 221A of the bioreactor 2, the cells CLS being of the type of cells CLS capable of forming an osteoarticular organoid;
- a step of feeding the cells CLS, called the feeding step 12, by using a nutrient fluid FLD providing each at least one growth well 221B, 221A to provide chemical and biological microenvironment to the cells CLS and the formation during the osteoarticular organoid, the feeding step 12 being carried out by a diffusion element 25 of the bioreactor 2;
- a step of mechanical stimulation of each at least one growth well 221B, 221A, called the stimulation step 13, to induce strain constraints on the deposition substrate 222, i.e. the 3D scaffold, seeded with cells CLS during organoid formation, the stimulation step 13 being carried out by a device for applying mechanical cyclic load of the bioreactor 2, called application device 27.

The feeding step 12 and the stimulation step 13 are achieved along a very long period of time, as for example during many days or several weeks and/or months. During this period, the feeding step 12 and/or the stimulation step 13 occurs continuously or intermittently. In a preferred embodiment of the method 1 according to the first aspect of the invention, the feeding step 12 is achieve continuously, as the cells CLS have to be fed with the nutrient fluid FLD to develop optimally, while the stimulation step 13 can be achieved intermittently, the stimulation step 13 of the method 1 comprising some activated stimulation periods during which the application device 27 induces mechanical stimulation to the cells CLS in each at least one growth well 221B, 221A, and some inactivated periods during which the application device 27 is turned off and/or does not apply mechanical stimulation to the said cells CLS.

The invention reveals that inducing a mechanical load over the growing period of the cells CLS into each growth well 221B, 221A is essential to produce osteoarticular organoids. Then, depending of several parameters of the nutrient fluid FLD used to feed the cells CLS during the feeding step 12 and/or the ambient conditions in which the cells CLS are cultivated and/or the parameters of the mechanical load applied to the cells CLS, it is possible to control the development and the type of osteoarticular organoids produced by such method 1 and such bioreactor 2. Those parameters will be described hereafter for a complete comprehension of the invention.

According to an important aspect of the invention, the method 1 should occur in a sterile environment. More precisely, the cells CLS developing in each growth well 221B, 221A should grow in a sterile environment. To achieve such sterile environment, the bioreactor 2 comprises a membrane 23 that is deposited on an upper surface 223 of a bioreactor 2's culture holder 22, on which culture holder 22 the growth well 221B, 221A is designed. As a sterile environment, one should understand that, for example, each growth well 221B, 221A is sealed by the membrane 23 in order to prevent impurities from entering each at least one growth well 221B, 221A and to better control the environmental conditions for cell CLS development.

Moreover, the temperature of the nutrient fluid FLD that is used to feed the cells CLS into each growth well 221B, 221A should be finely controlled in order to maintain a constant temperature during the feeding step 12 and, in a more general way, during the experiment. Advantageously, the working temperature should be equal or almost equal to 37°C. The working temperature is thus similar to the human body's one, which is optimistic for the fabrication of human osteoarticular organoids. To achieve such objective, the method 1 advantageously comprises a temperature regulation step 15. As an example, the temperature regulation step 15 is achieved with a temperature sensor associated with the nutrient fluid FLD itself and/or with each at least one growth well 221B, 221A. Moreover, the storage 31 in which the nutrient fluid FLD is stored in order to be transferred to the bioreactor 2 should include a heating system 32 in order to make it possible to heat said nutrient fluid FLD stored.

According to an embodiment of the invention, the cells CLS to be develop into each at least one growth well 221B, 221A of the bioreactor 2 should be placed in an hypoxic environment. In other words, the cell CLS's development into each growth well 221B, 221A should be achieved with an oxygen-poor atmosphere. Such atmosphere is more easily feasible thanks to the membrane 23 that isolates the growth well 221B, 221As from the other parts of the bioreactor 2. Thus, the oxygen concentration of the nutrient fluid FLD that is transported from the storage 31 to each growth well 221B, 221A of the bioreactor 2 is also regulated, thanks to an oxygen regulation step 14 of the method 1 according to the invention. The isolation of the growth well 221B, 221As from the other parts of the bioreactor 2 is very convenient to produce a small area in which such atmosphere is very well defined and very well controlled over long periods of time, as it's very important for the quality of the osteoarticular organoids to be fabricated.

The feeding step 12 is fundamental to produce different kind of osteoarticular organoids. As described before, the method 1 according to the first aspect of the invention makes it possible to produce a bone tissue type, and/or a cartilage type and/or an intervertebral disc type. Depending on the type of the osteoarticular organoid to be produced, the nutrient fluid FLD should be precisely defined. In a very general way, the nutrient fluid FLD should be of the type of a glucose-rich nutrient fluid FLD. The glucose concentration of the nutrient fluid FLD should be greater than 0,25 g/L. Moreover, in order to keep a very good quality of the nutrient fluid FLD, at least a quarter of the nutrient fluid FLD should be changed in the storage 31 when the glucose content is under the 0,25 g/L or the lactate greater than 1800 mg.L⁻¹. TheFLD stored is monitor continuously in hypoxic conditionsto ensure its quality.

Then, similarly, the stimulation step 13 has also to be adapted to the type of the osteoarticular organoid to be produced : for a soft osteoarticular organoid to be produced, such as an intervertebral disc for example, the stimulation step 13 should be larder, i.e. the magnitude of such mechanical strains induced by the application device 27 to the cells CLS should be greater than those for a hard osteoarticular organoid, such as a bone for example. In a very general way, the controlled relative strains induced by the application device 27 onto the growing cells CLS is in the range of 0,1% to 15%. Such strains are produced thanks to a vertical controlled displacement of upper face of the cells CLS in the range of 10 µm to 500 µm. Of course, in the case where the stimulation step 13 is achieved with an actuator 271 that produces a force onto the growing cells CLS through the displacement of a moving element of said actuator 271, the deformation aforementioned are induced by a displacement of the moving element in the range of 10 µm to 500 µm.

According to a first mode of realization of said method 1 according to the invention, here are described the parameters for producing an osteoarticular organoid of the type of a bone :
- the stimulation step 13 should apply, at the initiation of the protocol, a strain onto the deposition substrate 222 - i.e. into the 3D scaffold - seeded with cells CLS equal or almost equal to 4%, the strain value being reduced over time according to organoid stiffness;
- the stimulation step 13 should apply some repetitive compression efforts onto the cells CLS at a frequency equal or almost equal to 1 Hz and during an active period in the range of 30 min to 2 hours per day, during at least 3 weeks, preferably at least 2 months;
- the cells CLS should be put in a sterile environment;
- the cells CLS should be put in an hypoxic environment, said method 1 comprising an oxygen regulation step 14 providing an oxygen concentration in such sterile environment in the range of 5% to 7%;
- the nutrient fluid FLD chosen between the osteogenic differentiation mediums.

According to a second mode of realization of said method 1 according to the invention, here are described the parameters for producing a soft osteoarticular organoid of the type of a cartilage :
- the stimulation step 13 should apply, at the initiation of the protocol, a relative deformation of the growing cells CLS equal or almost equal to 10%, the relative deformation value being reduced over time according to organoid stiffness;
- the stimulation step 13 should apply some repetitive compression strength onto the cells CLS at a frequency equal or almost equal to 1 Hz and during an active period in the range of 30 min to 2 hours per day, during at least 3 weeks, preferably at least 2 months;
- the cells CLS should be put in a sterile environment;
- the cells CLS should be put in an hypoxic environment, said method 1 comprising an oxygen regulation step 14 providing a null oxygen concentration in such sterile environment;
- the nutrient fluid FLD chosen between the chondrogenic differentiation mediums.

According to a third mode of realization of said method 1 according to the invention, here are described the parameters for producing an osteoarticular organoid of the type of an intervertebral disc:
- the stimulation step 13 should apply, at the initiation of the protocol, a relative deformation of the growing cells CLS greater than 10%, the relative deformation value being reduced over time according to organoid stiffness;
- the stimulation step 13 should apply some repetitive compression efforts onto the cells CLS at a frequency equal or almost equal to 1 Hz and during an active period in the range of 30 min to 2 hours per day, during at least 3 weeks, preferably at least 2 months;
- the cells CLS should be put in a sterile environment;
- the cells CLS should be put in an hypoxic environment, said method 1 comprising an oxygen regulation step 14 providing an oxygen concentration in such sterile environment in the range of 5% to 7%;
- the nutrient fluid FLD chosen between the XXX differentiation mediums.

The method 1 according to the invention is implemented by means of a bioreactor 2, an example of which being described hereafter. Such bioreactor 2 comprises:
- a frame 21 that providing a controlled and delimited environment for the cell CLS's development, and more specially the sterile and temperature and/or oxygen-controlled environment. As illustrated in FIGURE 2, the frame 21 as an elongated shape, for example a cylindrical shape, and it delimits an inner cavity 212 in which the experiment is conducted. To his purpose, the frame 21 is proposed with non-oxidable materials, such as metallic materials for example, as stainless steel for example;
- a culture holder 22 housed in the frame 21, the culture holder 22 comprising at least one growth well 221B, 221A forming a cavity arranged on the support, each at least one growth well 221B, 221A delimiting a deposition substrate 222 for cells CLS intended to be produced to form the osteoarticular organoid. The culture holder 22 is a kind of sample holder to produce cells CLS. The culture holder 22 is attached to the frame 21, and the culture holder 22 is configured to be set inside the frame 21, in the inner cavity 212;
- an element for diffusing a nutrient fluid FLD in each at least one growth well 221B, 221A, called diffusion element 25. The diffusion element 25 is configured to organize the nutrient fluid FLD distribution for each growth well 221B, 221A. In other words, the diffusion element 25 is configured to allow the nutrient fluid FLD flow from the storage 31 to each growth well 221B, 221A;
- a device for applying mechanical load to each at least one growth well 221B, 221A, called application device 27, said application device 27 being configured to generate an axial force directly on the upper surface of the scaffold seeded with cells 222;
- a control unit 30 configured to drive the application device 27 and the diffusion element 25.

As visible in the schematic of FIGURE 2, the frame 21 has a general cylindrical conformation along a specific elongation axis X, the culture holder 22 being housed axially in the frame 21. The frame 21 comprises a base 211 at its bottom, on which base 211 a compression sensor 28 is attached in order to measure - through the application device 27 - the applied strength by said application device 27 to each cells CLS into each growth well 221B, 221A. At the opposite end regarding the elongation axis X, the frame 21 has an open-shape, providing a large aperture through which the culture holder 22 can be inserted into the inner cavity 212 of the frame 21. The frame 21 also comprises some retaining elements for the culture holder 22, inside the inner cavity 212, non represented in FIGURE 2.

The culture holder 22 has a cylindrical shape which is compatible with the inner cavity 212 of the frame 21. The culture holder 22 is removable from the inner cavity 212 of the frame 21, to makes it easier to deposit the cells CLS into each growth well 221B, 221A. The culture holder 22 Is preferably made with autoclavable polymer material. Suxh as PEEK On its upper surface 223, i.e. the one that faces the large upper aperture of the frame 21, the culture holder 22 delimits at least one growth well 221B, 221A.

Each growth well 221B, 221A has a concave shape to retains the cells CLS that are developing during the application of the method 1 according to the invention. In particular, each growth well 221B, 221A is a hole made from the upper surface 223 of the culture holder 22, such hole elongates along the elongation axis X. In the context of the present invention, the culture holder 22 may propose one or several growth well 221B, 221As in order to produce one or several osteoarticular organoids. If the culture holder 22 holds several growth well 221B, 221A, every grow wells are located onto the upper surface 223 of the culture holder 22 ; and they're organize in any way onto said upper surface 223.

Each growth cell CLS as a flat lower surface, said lower surface being a deposition substrate 222 for the scaffold seeded with cells CLS to be develop. In a preferred embodiment, the deposition substrate 222 of each growth well 221B, 221A are at the same level, regarding the elongation axis X, but each growth well 221B, 221A could also present different depths.

Typical dimensions of growth well 221B, 221As can be variable, depending on the size of osteoarticular organoids to be produced. Typically, the growth well 221B, 221A diameter is the range of 6 mm, advantageously included between 2 mm and 15 mm. The growth well 221B, 221A depth - measured along the elongation axis X - is in the range of few millimetres, for example between 4 mm and 10 mm.

The diffusion element 25 advantageously comprises a diffusion channel 251 arranged in the culture holder 22 and opening onto each at least one growth well 221B, 221A. The diffusion channel 251 has a groove shape connecting each growth well 221B, 221A. The diffusion channel 251 is designed from the upper surface 223 of the culture holder 22. The diffusion channel 251 opens onto the deposition substrate 222 of each growth well 221B, 221A, in order to make it possible for the nutrient fluid FLD that flows into the diffusion channel 251 to flow toward and onto each cells CLS deposited onto said deposition substrate 222. The deposition substrate 222 is for example of a 3D scaffold type.

The diffusion channel 251 is designed from a lateral edge of the culture holder 22, toward each growth well 221B, 221A. The culture holder 22 may also comprise some coupling elements in order to provide a sealed connexion between the diffusion channel 251 and some flexibles located outside from the frame 21 and used for the transportation of said nutrient fluid FLD.

In the case where the culture holder 22 comprises a plurality of growth well 221B, 221As, then the diffusion channel 251 connects each growth well 221B, 221A in a serial or star configuration. Moreover, in such configuration, it is advantageous that a first segment of the diffusion channel 251, taken before a first growth well 221B, 221A, being lower, i.e. less depth, than a second segment of said diffusion channel 251, taken after the first growth well 221B, 221A and before a second growth well 221B, 221A, said second growth well 221B, 221A following the first growth well 221B, 221A. This advantageous configuration optimizes the nutrient fluid FLD flow along the diffusion channel 251 and along each growth well 221B, 221A.

To secure a sterile environment in each growth well 221B, 221A, the bioreactor 2 advantageously comprises a membrane 23 for sealing the culture holder 22, the membrane 23 being configured to overly on each growth well 221B, 221A and the diffusion channel 251, to seal the culture holder 22. The membrane 23 is associated with the upper surface 223 of culture holder 22. The membrane 23 is flexible along the elongation axis X of the frame 21. Thus, the membrane 23 extends onto each growth well 221B, 221A and above each cells CLS. As a non limitative example, the membrane 23 is of the silicone membrane 23 type.

As an important feature of the invention, the membrane 23 should contact the cells CLS in order to transmit the pressure that is about to be applied by the application device 27 on said membrane 23, to the cells CLS themselves. Then, the bioreactor 2 comprises at least one levelling pellet arranged in each at least one growth well 221B, 221A. Each levelling pellet is associated with the cells CLS inside the growth well 221B, 221A. The levelling pellet is arranged onto each cells CLS in order to compensate the distance remaining between the cells CLS and the membrane 23, i.e. between the cells CLS and the culture holder 22's upper surface 223 if the membrane 23 is not used.

As describer before, the application device 27 is dedicated to applying a force onto each cells CLS into each growth well 221B, 221A. The force is applied along the elongation axis X to define and control the mechanical load applied on cells CLS. To provide such force, the application device 27 is advantageously a displacement actuator 271. The free extremity of such actuator 271 is movable along the elongation axis X and induce the cell CLS's compression.

The application device 27 is disposed on the upper part of the frame 21. More precisely, the application device 27 is attached to a cover 26 that extends across the upper aperture of the frame 21. Thus, the application devise is disposed upwardly in the inner cavity 212 and faces the culture holder 22.

In the context of the invention, the application device 27 can be of any type, is pneumatic, hydraulic, electric or piezoelectric. The application device 27 is controlled by the control unit 30 that is configured to control the displacement, the speed and the frequence of said application device 27, as such displacements are of the type of oscillating displacements.

As visible on FIGURE 2, the application device 27 is associated to an adapter 24 that extends between the lower extremity of the actuator 271 to the culture holder 22. The adapter 24 extends into the inner cavity 212 of the frame 21. On an upper part of the adapter 24, said adapter 24 comprises a connexion part 241 with the actuator 271. The connexion part 241 is coupled with the actuator 271's free extremity thanks to connecting means that are non-visible on FIGURE 2. From the connexion part 241, the adapter 24 comprises :
- a guiding beam 243 that extends from the connexion part 241 and along the elongation axis X. The guiding beam 243 extends in a central part of the adapter 24 and through the culture holder 22. To this purpose, the culture holder 22 comprises an central aperture whose shape and dimensions are compatible with those of the guiding beam 243, in order to make it possible for the guiding beam 243 to translate without clearance or with minimum clearance. This advantageous configuration makes it possible to constrain the adapter 24's displacement to a very linear translation that is parallel to the elongation axis X. Moreover, the frame 21 may also include such central aperture - , called cylindrical opening 213 - in order to offer such guiding capabilities. By guiding the adapter 24 along the elongation axis X, it's thus possible to equally distribute the force induced by such displacement to each growth well 221B, 221A and each cells CLS located into said growth well 221B, 221A, in the case where the culture holder 22 comprise more than one growth well 221B, 221A ;
- at least one terminal finger 242B 242A that extends from the connexion part 241 and along the elongation axis X. The at least one terminal finger 242B 242A extends peripherally from the guiding beam 243. In a preferred embodiment, the adapter 24 comprises several terminal finger 242B 242A, each terminal finger 242B 242A being associated with one growth well 221B, 221A. More precisely, each terminal finger 242B 242A extends above one growth well 221B, 221A, in order to be in contact with the membrane 23 and/or with the levelling pellet and/or with the cells CLS while the application device 27 is pushing against the growth well 221B, 221A deposition substrate 222. Thus, the mechanical constraints are applied to the cells CLS by a structural link of elements that are connected or temporally associated together through some axial contacts regarding the elongation axis X. This advantageous configuration makes it possible to transfer efforts from the actuator 271 to the cells CLS themselves through the adapter 24, the membrane 23 and the levelling pellet.

Advantageously, the bioreactor 2 also comprises a spring device 281 that is located oppositely to the force generator and that is suitable to provide an opposite force to whom provided by said force generator. The spring device 281 is attached to the frame 21 and collaborates with the adapter 24 and its free end 244. The spring device provides a force that is parallel with the elongation axis X. the spring device allows the adapter 24 and the force generator to retract upwardly from the cells CLS and the culture holder 22.

The adapter. 24 is made with a non-oxidable material, such as stainless steel form example.

To finely control the constraints applied to the cells CLS, the bioreactors also includes a displacement sensor 29 and a compression sensor 28.

The displacement sensor can be of any type. As an example, it's preferably of the type of an optical device. In the example schematically illustrated in FIGURE 2, the displacement sensor 29 is a laser telemeter. The displacement sensor 29 is attached to the frame 21 and measures the displacement imposed by the actuator 271 and/or the adapter 24 relatively to the elongation axis X. The displacement sensor 29 is attached outwardly from the inner cavity 212. To this purpose, the adapter 24 advantageously comprises a lateral flange 245 that extends radially from the frame 21. As the adapter 24 is moving along the elongation axis X, following the actuator 271's displacement, the lateral flange 245 is moving upward or downward, said position being measured by the displacement sensor 29. The frame 21 includes a lateral aperture 214 through which the lateral flange 245 extends.

The compression sensor is attached to the frame 21's based, and measures the force applied by a free end 244 of the guiding beam 243 onto said compression sensor 28. Thus, as the adapter 24 is moving along the elongation axis X, following the actuator 271's displacement, the free end 244 is moving upward or downward, pushing or realising the compression sensor 28 that measures a force applied by the adapter 24 on the top of it. Of course, the force measurement achieved by the compression sensor 28 is relevant with the force applied by the actuator 271 on each cell CLS in each growth well 221B, 221A.

Thus, by computing the ratio between the force measurement and the displacement measurement, it is possible to estimate the stiffness of each cells CLS in each growth well 221B, 221A. In other words, the stiffness of the organoid is known for the duration of the culture, making it possible to determine the displacement that actuator 271 must impose on each cell CLS in each growth well 221B, 221A.

To finish, as visible in FIGURE 2, the invention also addresses a system for producing an osteoarticular organoid, and the culture system 3 comprising:
- the bioreactor 2 as describer here before;
- a nutrient fluid FLD storage 31, the nutrient fluid FLD storage 31 being connected to the diffusion element 25 of the bioreactor 2 by fluid conduits;
- a pump 33 for controlling a flow rate of the nutrient fluid FLD in the diffusion element 25 of the bioreactor 2;
- an heating system 32 configured to regulate a temperature of the nutrient fluid FLD stored in the nutrient fluid FLD storage 31 at a set temperature, preferably equal to or substantially equal to 37°;
- a hypoxia oven for controlling an oxygen concentration in the bioreactor 2 and in particular at the diffusion element 25;
- a control unit 30 configured to control the culture system 3, the control unit 30 being interfaced with the control unit 30 of the bioreactor 2.

In summary, the invention concerns a method 1 for producing osteoarticular organoids with a bioreactor 2, the method 1 comprising a mechanical stimulation step 13 that occurs during a feeding step 12. While the feeding step 12 feeds the cells CLS with a nutrient fluid FLD, the stimulation step 13 provide cyclic compressions to the cells CLS, in order to activate the mechanical characteristics sought for such osteoarticular organoids depending on its own stiffness. The production occurs in a sterile environment and with hypoxic conditions, said metho comprising an oxygen regulation step 14 and a temperature regulation step 15 to finely control the temperature at 37°C during the production phase, i.e. more than 1 month.

Of course, the invention is not limited to the examples that have been described and many adjustments can be made to these examples without departing from the scope of the invention. In particular, the different characteristics, forms, alternatives and embodiments of the invention may be combined with each other in various combinations insofar as they are not incompatible or exclusive of each other. In particular, all the alternatives and embodiments described hereabove can be combined with each other.

## Claims

1. A method (1) for producing osteoarticular organoids using a bioreactor (2), the production method (1) comprising the following steps:
- a step of depositing cells (CLS) onto a deposition substrate (222) of the bioreactor, such as a scaffold, called the seeding step (11), in at least one growth well (221B, 221A) of the bioreactor (2), the cells (CLS) being of the type of cells (CLS) capable of forming an osteoarticular organoid;
- a step of feeding the cells (CLS), called the feeding step (12), by using a nutrient fluid (FLD) providing each at least one growth well (221B, 221A) in order to provide a chemical microenvironment to the cells (CLS) during the formation of the osteoarticular organoid, the feeding step (12) being carried out by a diffusion element (25) of the bioreactor (2);
- a step of mechanical stimulation of each at least one growth well (221B, 221A), called the stimulation step (13), in order to induce strain constraints flet by the cells (CLS) during organoid formation, the stimulation step (13) being carried out by a device for applying mechanical constraints of the bioreactor (2), called application device (27).

2. Method (1) according to the previous claim, in which the feeding step (12) and/or the stimulation step (13) are carried out under hypoxic conditions and in a sterile environment.

3. Method (1) according to any one of the previous claims, in which the feeding step (12) is carried out continuously and the stimulation step (13) is carried out during said feeding step (12), said stimulation step (13) being carried out during one or more cycles of said feeding step (12), called stimulation cycles.

4. Method (1) according to any one of the previous claims, in which the stimulation step (13) generates several series of mechanical load applied to each at least one growth well (221B, 221A), the mechanical load aiming to impose repeated deformation or forces on the cells (CLS) and/or on the deposition substrate (222) during organoid formation, the mechanical load produced during said stimulation step (13) being configured to generate on the cells (CLS) of each at least one growth well (221B, 221A) an initial deformation at least equal to 10%.

5. Method (1) according to any one of the previous claims, in which the mechanical constraints carried out during the stimulation step (13) are carried out at a determined frequency, advantageously included between 0.5 Hz and 10 Hz, preferably between 0.5 Hz and 5 Hz, more preferably equal or substantially equal to 1 Hz.

6. Method (1) according to any one of the previous claims, in which the method (1) comprises a step of regulating a temperature of the nutrient fluid (FLD), called the temperature regulation step (15), to a predetermined set temperature, preferably equal to or substantially equal to 37°C.

7. Bioreactor (2) for producing an osteoarticular organoid, the bioreactor (2) comprising means configured to implement the production method (1) according to any one of the preceding claims.

8. Bioreactor (2) according to the preceding claim, in which the means comprise:
- a frame (21);
- a culture holder (22) housed in the frame (21), the culture holder (22) comprising at least one growth well (221B, 221A) forming a cavity arranged on the culture holder (22) itself, each at least one growth well (221B, 221A) delimiting a deposition substrate (222) - as well as a 3D scaffold for example - for cells (CLS) intended to be produced in order to form the osteoarticular organoid;
- an element for diffusing a nutrient fluid (FLD) in each at least one growth well (221B, 221A), called diffusion element (25);
- a device for applying mechanical load to each at least one growth well (221B, 221A), called application device (27), the application device (27) being configured to generate an axial force directly above each deposition substrate (222);
- a control unit (30) configured to drive the application device (27) and the diffusion element (25).

9. Bioreactor (2) according to claim 9, in which the nutrient fluid (FLD) diffusion element (25) comprises a diffusion channel (251) arranged in the culture holder (22) and opening onto each at least one growth well (221B, 221A).

10. Bioreactor (2) according to any one of claims 8 or 9, in which the bioreactor (2) comprises a membrane (23) for sealing the culture holder (22), the membrane (23) being configured to seal the culture holder (22) in a sealed manner, i.e. to seal the at least one growth well (221B, 221A) and the diffusion channel (251), the membrane (23) being deformable along the specific elongation axis (X) of the frame (21).

11. Bioreactor (2) according to any one of claims 8 to 10, in which the application device (27) comprises:
- a force generator configured to generate an axial force on the deposition substrate (222) of each at least one growth well (221B, 221A);
- a compression sensor (28) configured to determine the axial force applied by the force generator on the deposition substrate (222) of each at least one growth well (221B, 221A), the control unit (30) controlling the application device (27) according to the axial force measured by the compression sensor (28).

12. Bioreactor (2) according to the preceding claim, in which the application device (27) comprises an adapter (24) coupled to a free end (244) of the force generator, the adapter (24) being configured to transmit the force produced by the axial movement of the force generator to each at least one growth well (221B, 221A), a free end (244) of the adapter (24) being associated with each produced cell (CLS), the adapter (24) comprising :
- a translational guiding beam (243) along the elongation axis (X) of the frame (21), the guiding beam (243) collaborating by sliding in a cylindrical opening (213) provided in the frame (21) ;
- at least one terminal finger (242B) (242A), each at least one terminal finger (242B) (242A) being associated with one of the at least one growth well (221B, 221A) so as to transmit the force produced by the actuator (271).

13. Bioreactor (2) according to any one of claims 8 to12, in which the bioreactor (2) comprises a displacement sensor (29) of the adapter (24), the displacement sensor (29) being attached with the frame (21).

14. System (3) for producing an osteoarticular organoid, said culture system (3) comprising:
- the bioreactor (2) according to any one of claims 8 to 13;
- a nutrient fluid (FLD) storage (31), the nutrient fluid (FLD) storage (31) being connected to the diffusion element (25) of the bioreactor (2) by fluid conduits;
- a pump (33) for controlling a flow rate of the nutrient fluid (FLD) in the diffusion element (25) of the bioreactor (2);
- an heating system (32) configured to regulate a temperature of the nutrient fluid (FLD) stored in the nutrient fluid (FLD) storage (31) at a set temperature, preferably equal to or substantially equal to 37°;
- a hypoxia oven for controlling an oxygen concentration in the bioreactor (2) and in particular at the diffusion element (25);
- a control unit (30) configured to control the culture system (3), the control unit (30) being interfaced with the control unit (30) of the bioreactor (2).]
